# FASCICULE DE BREVET EUROPEEN

(11) **EP 0 615 544 B1**
(45) Date de publication et mention de la délivrance du brevet: **27.02.2002**
(21) Numéro de dépôt: 92923846.7
(22) Date de dépôt: 20.10.1992
(51) Int. Cl.: C12N 1/16, C12P 7/06

(54) **UTILISATION DES ANTIBIOTIQUES IONOPHORES POLYETHERS POUR LIMITER LA CROISSANCE BACTERIENNE EN FERMENTATION ALCOOLIQUE INDUSTRIELLE**
VERWENDUNG VON POLYETHERIN-IONOPHOREN ANTIBIOTIKA ZUR EINSCHRÄNKUNG DES BAKTERIELLEN WACHSTUMS IN INDUSTRIELLEN ALKOHOLISCHEN GÄRUNGEN
USE OF IONOPHORETIC POLYETHER ANTIBIOTICS FOR CONTROLLING BACTERIAL GROWTH IN INDUSTRIAL ALCOHOLIC FERMENTATION

(30) Priorité: 18.11.1991 FR 9114176
(43) Date de publication de la demande: 21.09.1994
(73) Titulaire: UNION NATIONALE DES GROUPEMENTS DE DISTILLATEURS D'ALCOOL (U.N.G.D.A.), 92247 Malakoff Cédex (FR)
(72) Inventeur: DE MINIAC, Michel, F-75015 Paris (FR)
(74) Mandataire: Ahner, Francis
(86) Numéro de dépôt international: FR9200984
(87) Numéro de publication internationale: WO9310213

(56) Documents cités:
- FR-A- 2 032 598
- FR-A- 2 587 035
- INTERNATIONAL JOURNAL OF FOOD MICROBIOLOGY vol. 3, no. 3, 1986, NL pages 135 - 141 S. HENNING ET AL. 'New aspects for the application of nisin to food products based on its mode of action'
- JOURNAL OF ANIMAL SCIENCE vol. 65, no. 4, 1987, US pages 1064 - 1076 T.G. NAGARAJA ET AL. 'In-vitro lactic acid inhibition and alterations in volatile fatty acid production by antimicrobial feed additives'
- JOURNAL OF APPLIED BACTERIOLOGY vol. 60, no. 6, Juin 1986, UK pages 491 - 500 J.D. PHILLIPS ET AL. 'Estimation of Gram-negative bacteria in milk. A comparison of inhibitor systems for preventing Gram-positive bacterial growth'
- CHEMICAL ABSTRACTS, vol. 97, no. 23, 6 Décembre 1982, Columbus, Ohio, US; abstract no. 197321n, F. KUTAS ET AL. 'Effect of monensin on rumen acidosis caused by lactic acid fermentation' page 496 ;colonne 2 ;

## Description

La présente invention concerne l'utilisation d'un quelconque des antibiotiques ionophores polyéthers pour limiter la croissance bactérienne dans les milieux de fermentation alcoolique industrielle, en particulier les milieux suivants : jus de betterave ou de canne à sucre, mélasses de betterave ou de canne à sucre, hydrolysats d'amidon, en particulier de céréales ou de tubercules, milieux fermentés d'origine vinique ou cidricole.

Dans l'état actuel de fonctionnement des ateliers, la flore bactérienne passe de 10⁴ germes/ml à 10⁶ germes/ml durant le procédé de fermentation alcoolique. A cette concentration de 10⁶ germes/ml, il y a production d'acides organiques qui gênent la croissance et la fermentation alcoolique de la levure. Cette flore bactérienne d'origine lactique peut également transformer le glycérol en acroléine dans les milieux d'origine vinique ou cidricole. L'acroléine est une substance cancérigène qui se retrouve par la suite dans l'alcool distillé que l'on destine à la consommation humaine.

Le problème de la contamination bactérienne dans les milieux de fermentation alcoolique industrielle pour la préparation d'alcool distillé est notamment décrit dans les demandes de brevet FR-2 587 035 et FR-2 032 598. L'ajout d'antibiotiques tels que la nisine ou la virginiamycine pour limiter ou réduire la croissance bactérienne dans les milieux fermentaires est notamment décrit par Herning & al. (International Journal of Food Microbiology, 1986, 3, 135-141).

La présente invention consiste à introduire dans le milieu fermentaire (moût sucré initial) une concentration proche de 0,5 ppm d'un antibiotique ionophore polyéther, qui a ici une action bactériostatique, c'est-à-dire qu'il empêche les bactéries de se développer, mais est sans action sur la levure jusqu'à une concentration de 100 ppm. Cette flore bactérienne maintenue à 10⁴ germes/ml ne produit aucune acidité organique et ne peut donc ralentir la fermentation alcoolique. Dans ces conditions, elle ne peut non plus produire d'acroléine. A des doses supérieures à 0,5 ppm d'antibiotique, celui-ci a une action bactéricide, et permet donc une décroissance bactérienne.

Les antibiotiques ionophores polyéthers constituent une classe bien définie d'antibiotiques employés notamment en thérapie vétérinaire, et comprennent notamment la monensine, le lasalocid, la maduramycine, la naraxine, la salinomycine ou encore la maversine (tous cités dans le "Merck Index" 11ème édition). Certains ont fait l'objet de demandes de brevets (EP-0 112 517, EP-0 169 011, EP-0 255 375, EP-0 385 594). Leur mode d'action et leur emploi comme facteur de croissance pour les ruminants ou comme médicament anticoccidien dans l'élevage aviaire industriel est en particulier décrit dans un rapport de thèse de Mme GABOYARD intitulé "ETUDE DE DERIVES DES ANTIBIOTIQUES IONOPHORES MONENSINE, LONOMYCINE, CATIOMYCINE. COMPLEXATION DE Na⁺ ET K⁺, TOXICITE, ACTIVITES ANTICOCCIDIENNE ET FACTEUR DE CROISSANCE" (Thèse présentée à l'Université de Clermont-Ferrand II le 30 janvier 1987).

L'adjonction d'un antibiotique ionophore polyéther dans le milieu fermentaire permet donc une meilleure régularité de fonctionnement des ateliers de fermentation alcoolique industrielle, ainsi qu'une meilleure qualité chimique de l'alcool distillé.

## Revendications

1. Utilisation d'un antibiotique ionophore polyéther à une concentration proche de 0,5 ppm dans les milieux de fermentation alcoolique industrielle afin d'empêcher la croissance des bactéries, sans avoir d'action sur la levure.

2. Utilisation selon la revendication 1, **caractérisée en ce que** les milieux de fermentation alcoolique industrielle sont choisis parmi les milieux suivants : jus de betterave ou de canne à sucre, mélasses de betterave ou de canne à sucre, hydrolysats d'amidon, en particulier de céréales ou de tubercules, milieux fermentés d'origine vinique ou cidricole.

3. Procédé industriel de préparation d'alcool distillé que l'on destine à la consommation humaine, comprenant la fermentation d'un milieu fermentaire et la distillation de l'alcool, **caractérisé en ce que** l'on introduit dans le milieu fermentaire un antibiotique ionophorc polyéther à une concentration proche de 0,5 ppm, afin d'empêcher la croissance des bactéries, sans avoir d'action sur la levure.

4. Procédé selon la revendication 3, **caractérisé en ce que** les milieux de fermentation alcoolique industrielle sont choisis parmi les milieux suivants : jus de betterave ou de canne à sucre, mélasses de betterave ou de canne à sucre, hydrolysats d'amidon, en particulier de céréales ou de tubercules, milieux fermentés d'origine vinique ou cidricole.

## Claims

1. Use of a polyether ionophoric antibiotic at a concentration close to 0.5 ppm in industrial alcoholic fermentation media for the purpose of preventing the growth of bacteria, without having any effect on yeast.

2. Use according to Claim 1, **characterized in that** the industrial alcoholic fermentation media are selected from the following media: beetroot or sugar cane juice, beetroot or sugar cane molasses, starch hydrolysates, in particular from cereals or tubers, fermented media of wine or cider origin.

3. Industrial process for preparing distilled alcohol which is intended for human consumption, comprising fermenting a fermentative medium and distilling the alcohol, **characterized in that** a polyether ionophoric antibiotic is introduced into the fermentative medium at a concentration close to 0.5 ppm for the purpose of preventing the growth of bacteria, without having any effect on yeast.

4. Process according to Claim 3, **characterized in that** the industrial alcoholic fermentation media are selected from the following media: beetroot or sugar cane juice, beetroot or sugar cane molasses, starch hydrolysates, in particular from cereals or tubers, fermented media of wine or cider origin.

## Patentansprüche

1. Verwendung eines ionophoren Polyether-Antibiotikums bei einer Konzentration von etwa 0,5 ppm in Medien zur industriellen Alkoholgärung, um das Wachstum von Bakterien zu verhindern, ohne eine Einwirkung auf die Hefe zu haben.

2. Verwendung nach Anspruch 1, **dadurch gekennzeichnet, dass** die Medien zur industriellen Alkoholgärung ausgewählt sind aus den folgenden Medien: Zuckerrüben- oder Zuckerrohrsaft, Zuckerrüben- oder Zuckerrohrmelassen, Hydrolysaten von Stärke, insbesondere von Getreide oder Knollen, gegorenen Medien von Wein- oder Apfelwein-Herkunft.

3. Industrielles Verfahren zur Herstellung von destilliertem Alkohol, der für den menschlichen Verbrauch bestimmt ist, umfassend die Gärung eines Gärungsmediums und die Destillation des Alkohols, **dadurch gekennzeichnet, dass** man in das Gärungsmedium ein ionophores Polyether-Antibiotikum bei einer Konzentration von etwa 0,5 ppm einführt, um das Wachstum von Bakterien zu verhindern, ohne eine Einwirkung auf die Hefe zu haben.

4. Verfahren nach Anspruch 3, **dadurch gekennzeichnet, dass** die Medien zur industriellen Alkoholgärung ausgewählt sind aus den folgenden Medien: Zuckerrüben- oder Zuckerrohrsaft, Zuckerrüben- oder Zuckerrohrmelassen, Hydrolysaten von Stärke, insbesondere von Getreide oder von Knollen, gegorenen Medien von Wein- oder Apfelwein-Herkunft.
